# EUROPEAN PATENT SPECIFICATION

(11) **EP 1 618 791 B1**
(45) Date of publication and mention of the grant of the patent: **11.06.2014**
(21) Application number: 05108607.2
(22) Date of filing: 03.04.1998
(51) Int. Cl.: A23C 11/10, A23C 20/00, A23L 1/30, A23L 1/305, A23J 3/22, A23K 1/165, A23L 1/314, A23J 3/16, A23J 3/34, A23K 1/16

(54) **High Beta-Conglycinin products and their use**
Produkte mit hohem Beta-Conglycinin-Gehalt und ihre Verwendung
Produits a forte teneur en beta-conglycinine et leurs utilisations

(30) Priority: 04.04.1997 US 42643
(43) Date of publication of application: 25.01.2006
(62) Divisional of application: 98915251.7
(73) Proprietor: Monsanto Technology, LLC, St. Louis, MO 63167 (US)
(72) Inventor: Bringe, Neal A., 63304, Saint Charles (US)
(74) Representative: Von Kreisler Selting Werner - Partnerschaft von Patentanwälten und Rechtsanwälten mbB

(56) References cited:
- EP-A- 0 072 617
- EP-A- 0 797 928
- US-A- 4 368 151

## Description

### Background of the Invention

The present invention relates to a high beta-conglycinin protein isolate,

Glycinin and beta-conglycinin account for approximately 70% of the proteins in soybeans. It has been postulated that the functional properties of soy protein ingredients in food systems can be improved by modifying the ratio of these proteins. Previous attempts have been to increase the ratio of glycinin to beta-conglycinin to improve the yield and quality of tofu-type soybean gels and to improve the content of sulfur amino acids for nutritional purposes [Kitamura, K. Trends Food Science & Technology 4:64-67, (1993), Murphy, P., et al., Food Technology 51:86-88, 110 (1997].

Dietary proteins are needed to replace metabolic losses of tissue and organ proteins, to form and deposit protein in new tissues and to replenish tissue loss as a consequence of pathological conditions. These needs are met by indispensable (essential) amino acids and dispensable amino acids that comprise dietary proteins. It is largely in this context that the nutritional value of dietary proteins is defined as the ability to meet daily requirements for essential amino acids (Steinke, F. et al. New Protein Foods in Human Health: Nutrition Prevention and Therapy, CRC Press, 1992). High quality proteins contain all the essential amino acids at levels greater than reference levels and are highly digestible so that the amino acids are available. In this context, egg white and milk proteins are the standards to which other proteins are evaluated and plant proteins are considered to have inferior nutritional value. The essential amino acids whose concentrations in a protein are below the levels of a reference protein are termed limiting amino acids, e.g., the sum of cysteine and methionine are limiting in soybeans.

Glycinin contains 3 to 4 times more cysteine and methionine per unit protein than beta-conglycinin (Fukushima D., Food Rev. Int. 7:323-351, 1991). Thus it is expected that an increase in the content of glycinin and a decrease in the content of beta-conglycinin results in enhanced protein quality (Kitamura, K. Trends Food Science & Technology 4:64-67, 1993; Kitamura, K., JARQ 29:1-8, 1995). This is consistent with the finding that the mean value of the sulfur-containing amino acid contents in the seeds of four representative lines which were low in beta-conglycinin was about 20% higher than that of four ordinary varieties (Ogawa, T. Japan. J. Breed. 39:137-147, 1989). A positive correlation was also reported between the glycinin:beta-conglycinin ratio (1.7-4.9) and the methionine or cysteine concentration of total protein, among wild soybeans (Kwanyuen et al., JAOCS 74:983-987, 1997). There are no reports of the amino acid composition of high beta-conglycinin lines (glycinin:beta-conglycinin ratio less than 0.25).

In addition to the ability of proteins to meet the body's daily needs for essential amino acids, dietary proteins can also contribute bioactive peptides and amino acid patterns which can reduce the risk factors for cardiovascular diseases, cancer and osteoporosis. These compositional factors should also be considered in accessing protein quality, especially in countries such as the United States where people on the average consume a large excess of dietary protein. Researchers (Sugano, et al. PCT No. WO89/01495; Sugano, M. J. Nutr 120:977-985,1990; Sugano. M. & Kobak, K. Annu. NY Acad. Sci. 676:215-222, 1993; Wang, M. J Nutr. Sci. Vitaminol. 41:187-195, 1995) have identified a pepsin-resistant fraction of soybean protein (5,000-10,000 molecular weight) that represents about 15% of the protein in isolated soy protein. Humans fed a diet with the pepsin-resistant fraction at 24 g or 48 g per day had lower LDL-Cholesterol and more fecal neutral and acidic steroid excretion than those fed diets with isolated soy protein or casein. The soy proteins which contribute to this pepsin-resistant fraction were not identified. Purified beta-conglycinin is more pepsin-resistant than purified glycinin (Astwood, J. & Fuchs, R. In Monographs in Allergy, Sixth International Symposium on Immunological and Clinical Problems of Food Allergy, Ortolani, C. and Wuthrich, B. editors, Basel, Karger, 1996), so it is disclosed that beta-conglycinin may be a primary contributor to the bioactive fraction. This possibility has not been demonstrated yet in a feeding study, or with protein made from soybeans having altered protein compositions.

The alpha and alpha-prime subunits of beta-conglycinin specifically interact with membrane components of human and animal liver cells in tissue culture experiments. The beta-conglycinin subunits are then incorporated, degraded and increase maximal binding of LDL to high-affinity receptors. It is proposed that such a mechanism could be responsible for the cholesterol lowering properties of soy protein isolates. However, it is not clear if significant amounts of dietary soy proteins can get to the liver in vivo. Lavarti et al. (J. Nutr. 122:1971-1978, 1992) reported on a study in which hypercholesterolemic rats were fed either glycinin or beta-conglycinin for two weeks. Both groups showed a 1/3 reduction in total serum cholesterol. There are no studies which determine the effects of soy protein isolates from soybeans with modified soy proteins compositions on the cholesterol lowering properties of soy protein isolate.

It is reasoned from Rhesus monkey studies using alcohol extracted (which removes isoflavones) and non-alcohol extracted soy protein isolate, that soybean isoflavones are the primary components of soy protein isolates responsible for the cholesterol lowering effects (Anthony, M.S., J. Nutr. 126:43-50, 1996). However, subjecting soy protein to ethanol extraction did not have any effect on its lipid-lowering effects in other studies using hamsters (Balmir et al., J. Nutr. 126:3046-3053, 1996) or rats (Topping et al., Nutr. Res. 22:513-520, 1980). Alcohol extraction processes can extract some proteins and can denature and aggregate the unique structures of soy proteins, likely affecting how they act in the GI tract. For example, Sugano et al., J. Nutr. 120:977-985, 1990 observed that methanol extraction completely eliminated the ability of high molecular weight soy protein peptides to bind and excrete steroids. Feeding isolated soy isoflavones (genistein and daidzein) had no flavorable effect on serum lipids or lipoproteins in humans (Colquhoun, et al., Atherosclerosis, 109:75, 1994).

The confusion about the relative roles of various soy protein isolate constituents in the observed cholesterol-lowering effects, are difficult to resolve by using processing technologies to create samples with altered composition. An improved approach is to specifically modify the components of interest in the soybeans.

An emerging key indicator for the risk of heart disease, is high serum homocysteine levels. Dietary methionine is a precursor to homocysteine, so a high consumption of methionine can potentially increase consumers risk of heart disease, especially if they also consume low levels of folic acid and vitamin B6 (McCully, K.S., The Homocysteine Revolution, Keats Publishing, Inc., New Canaan, Connecticut, 1997). Another route which lowers the endothelial cytoxicity of homocysteine is the reaction between nitric oxide (NO) and homocysteine in vivo to form the non-toxic S-nitroso-homocysteine. This route can be enhanced by increasing dietary arginine levels because arginine is converted by nitric oxide synthase to NO. Therefore, an ideal dietary protein for maintaining healthy levels of homocysteine, as disclosed in this invention, should have high arginine and low methionine (and cysteine), as is found in beta-conglycinin. However, prior to this disclosure, the use of a beta-conglycinin rich soy protein isolate designed for this purpose was not considered.

New protein ingredients must contribute positively to the taste, texture and appearance of foods to gain acceptance. These quality attributes are determined by the structure of the proteins and how they change in the presence of other food components (e.g., calcium ions, other proteins) and processing conditions (e.g., temperature, pH). Increasing the glycinin content of soybeans is usually proposed for improving food functionality of soy protein ingredients. Previous attempts to improve the yield and quality of tofu-type soybean gels have been to increase certain glycinins or the ratio of glycinin to beta-conglycinin (Wang, C-C. and Chang, S. J. Agric. Food Chem. 43:3029-3034, 1995; Yagasaki, K. et al. Breeding Sci. 46:11-15, 1996; Murphy, P., et al., Food Technology 51:86-88, 110, 1997). There is little information on the properties of glycinin and beta-conglycinin in other model food systems, especially under conditions typical of other food systems (e.g., low pH, high salt, fat, gel formation at temperatures below 72 degrees C). Foaming properties of glycinin are superior to those of beta-conglycinin at a pH of 7.0 and no salt (Yu, M.A., J. Agric. Food chem. 39:1563-1567, 1991). Partially hydrolyzed glycinin forms heat-induced gels which are more similar to cheese curd than partially hydrolyzed beta-conglycinin at neutral pH (Kamata et al., Nippon Shokuhin kogyo Gakkaishi 36:557-562, 1989). Glycinin forms gels at boiling temperature with higher elastic moduli than soy protein isolate (Van Kleef, Biopolymers 25:31-59, 1986). Some comparisons were made between glycinin and beta-conglycinin at pH 7.5-8.0 (Shimada, K. and Matsushita, S., Agric. Biol. Chem. 44:637-641, 1980; Utsumi, S. and Kinsella, J. Food Sci. 50:1278-1282, 1985; Nakamura et al., Agric. Biol. Chem. 50:2429-2435, 1986). Though beta-conglycinin was observed to have superior emulsifying properties compared to glycinin, it did not have better emulsifying properties compared to whole soy protein isolate controls (Aoki et al., J. Food Sci. 45:534-546, 1980; Yao et al. JAOCS 67:974-979, 1990). The freeze-thaw properties of beta-conglycinin and glycinin rich soy protein isolates have not been reported.

Soybean germplasm which lack glycinin, sib-line varieties B2W(2), B2G(2), and B2G(1) were received from Dr. Norihiko Kaizuma, President of Tohoku University, Morioka, Japan (10/7/96). The mutation of these soybean lines was induced by using gama-irradiation (Odanaka, H. and N. Kaizuma, Japan J. Breed. 39 (Suppl. ) 430-431, 1989; Kaizuma et al. Jpn J. breed. 40 (Supple 1) 504-505, 1990). These lines lack all of the group-I subunits consisting of A1aB2, A1bB1b and A2B1a. Synthesis of the missing polypeptides has been shown to be controlled by a single recessive allele. No deleterious effects on physiological aspects such as seed development and germination were observed.

The properties of high beta-conglycinin isolates at pH 7 were discussed in Nagano, T. J. Agric. Food Chem. 44:3484-3488. The gel-forming properties at 85 degrees C and foaming properties of enzymatically hydrolyzed beta-conglycinin fractions were discussed in Lehnhardt, W.F. and Orthoefer, F.T., European patent no. 0072617, 1982.

Yields of protein and other soybean constituents also need to be considered in designing a commercially viable variety. Positive correlations were found between total protein content of soybeans and the glycinin:beta-conglycinin ratio, so the soybeans that are richer in glycinin had a higher protein content (Shui-Ho Cheng, 1984 Ph.D. thesis, Univ. of IL).

### Summary of the Invention

The present invention relates to a soy protein isolate having a beta-conglycinin content greater than 40% of the protein and a glycinin content of less than 10% of the protein, wherein the sum of methionine and cysteine in the composition is greater than 24 mg/g protein (hereinafter shortly "high BC-SPI" or "BC-SPI of the invention"), and further wherein (a) the beta conglycinin lacks alpha and alpha'-subunits; or (b) the composition lacks one, two or three lipoxygenases. The infention further relates to the use of the above composition for (a) emsulsified meat; (b) a soy cheese analog; or (c) a liquid frozen coffee whitener. The method of the manufacturing the BC-SPI of the present invention comprises removing the hulls from soybean seeds and conditioning the seeds for flaking. The conditioned seeds are flaked and oil is extracted. The soybean seed flakes are then preferably ground and a solvent is added to bring the pH to a range from about 8.5 to about 10 to dissolve the protein. An extract is produced by removing the fiber by centrifuging. The extract is neutralized and the sugars and other low molecular weight solutes are removed. The resulting product is a slurry which is dried. The present invention also includes methods for using the BC-SPI as different food products.

### Description of the Invention

To provide an understanding of several of the terms used in the specification and claims, the following definitions are provided:
High beta-conglycinin soybeans: As used herein, high beta-conglycinin (BC) rich soybeans refers to soybean seeds which have BC greater than 40% and having glycinin with less than 10% of the protein dry weight using the methods defined in Example 1.
Soy protein isolate (SPI): As used herein, soy protein isolate is a spray-dried powder made from soybeans containing not less than 90% protein (N x 6.25) on a moisture-free basis.
High beta-conglycinin soy protein isolate: As used herein, high beta-conglycinin soy protein isolate (BC-SPI) refers to a spray-dried powder which is made from high BC soybean seeds. The amount of BC in BC-SPI is greater than 40% of the protein dry weight in the isolate and the amount of glycinin in BC-SPI is less than 10% of the protein dry weight in the isolate using the methods in Example 1.
Hunter L Valve: As used herein, the term "Hunter L Valve" is the measure of whiteness by a colorimeter.
Hunter B Valve: As used herein, the term "Hunter B Valve" is the measure of yellowness by a colorimeter.
Nitrogen Solubility Index (NSI): As used herein, the term "Nitrogen Solubility Index (NSI)" is the measure of protein solubility using Method Ba11-65 of the Official and tentative Methods of the AOCS, 1989, 4th Edition.
Partially Hydrolyzed: As used herein, the term "Partially Hydrolyzed" means the hydrolysis products of BC.
Emulsified Meats: As used herein, the term "Emulsified Meats" means soy containing meats having a tender texture, such as frankfurters and bologna.
Nutritional Food Bar: As used herein, the term "Nutritional Food Bar" means a food bar designed to promote health.
Mouthfeel: As used herein, the term "Mouthfeel" means how the substance feels in a human mouth.

The present invention reveals physiological as well as food functionality benefits of BC-SPI. The BC-SPI of the present invention has higher than expected levels of essential amino acids. The high BC-SPI of the present invention has improved properties for food processing and products.

### Assessing the value of proteins in fat-containing foods

Fat-containing food products such as frankfurters, processed cheese, salad dressings, sauces, and nutritional beverages depend on emulsifiers to help form very small fat droplets during homogenization processes and then stabilize the droplets against coalescence (fusing of droplets) and creaming (floating to the top) during storage. Proteins which are especially good emulsifiers have highly flexible structures which allow high affinity and adsorption of the proteins at the oil-water interfaces, followed by the ability to form mechanically strong and viscoelastic and highly hydrated films on the droplet surfaces via protein-protein interactions. In some products controlled aggregation of the protein-stabilized fat droplets following heating or enzymatic hydrolysis of proteins, is important for forming gel structures which hold water and provide texture to foods. Animal proteins such as caseins in milk, lipoproteins in egg yolk, myosins in meat and albumin proteins in egg white are good emulsifying agents which have both stabilizing and gel-forming properties (Bringe, N., In "Food Proteins and Lipids," Damodaran, S., ed., Plenum Press, NY, pp. 161-181, 1997). The opportunity of the instant invention is to replace animal proteins with less expensive and healthful soy protein ingredients.

The potential of new protein sources to replace animal proteins as food emulsifiers can be determined by measuring the diameter of protein-stabilized fat-droplets which are formed under conditions which break the fat particles into small droplets. A good protein emulsifier adsorbs to the new oil-water surfaces quickly and stabilizes the droplets from coalescence, resulting in emulsions having the smallest median particle diameters. Poor emulsifying proteins do not cover all of the new oil-water surfaces and have poorly hydrated structures which do not repulse (prevent aggregation with) other protein-covered droptets. The poor protein emulsifiers cause the formation of larger particles via droplet-droplet aggregation and the large particles rise in space with time leaving a serum layer at the bottom of suspensions. Large particle aggregates are also detected in the mouth as chalky or gritty textures. Small particles are not detected in the mouth as individual particles and create a smooth or creamy texture (Bringe, N. and Clark, D. In "Science for the Food Industry of the 21st Century", Yalpani, M. ed., ATL Press, pp. 51-68, 1993). To determine the potential of new protein ingredients to replace other ingredients, one can prepare emulsions with the ingredients under various conditions relevant to different foods and determine the sizes of the droplets formed and the amount of serum created after storage (Bringe, N. et al., J. Food Sci. 61:1-6, 1996).

### Beneficial functional properties of purified BC

Before high BC soybeans were available for testing, the properties of purified BC were compared to glycinin and commercial isolates in model food emulsions. In the instant invention we discovered that BC formed smaller emulsion particles than control and commercial soy protein ingredients when the emulsions were prepared in the presence of ionic sodium (or potassium) or ionic calcium at levels similar to that found in foods such as nutritional beverages as shown in Example 2 . The good emulsifying properties of BC are important for keeping beverage emulsions from separating (creaming).

Further aspects of the invention include the discoveries that BC performed better than control and commercial soy protein ingredients when the protein-stabilized emulsions were heat-treated or when the emulsions were frozen and thawed as described in Example 2. This property is valuable in applications such as frozen desserts and liquid frozen coffee whitener where the smooth homogenous textures and appearances of the products depends on the stability of the proteins against freeze-thaw-induced protein aggregation.

Another embodiment of the invention is that heat-induced gel or viscosity forming properties of BC stabilized emulsions were optimum near pH 5.6 and significantly greater than control and other soy protein ingredients in the presence of salt as described in Example 2, Table 3. The preparation of food gels from BC-stabilized emulsions in the pH range 5.4 to 5.8 and low salt concentrations (0.2-0.6% NaCl or KCI) can now be conceived and designed under this invention. The gelling property is in the pH region of emulsified meats where soy protein ingredients are used as gelling agents. The limitation of the discovery for meat applications was that meat systems are not heated above 71 degrees C and contain at least 3.5% NaCl in the water phase. These conditions are included in the example below.

### BC-SPI

To take advantage of the properties of BC in the above applications, an economical means of preparing a SPI which is rich in BC and lacking in glycinin was developed in the present invention. The use of high BC soybeans which contain less than 10% glycinin and more than 40% BC, enable the preparation of BC-SPI without the inefficiencies of removing glycinins during processing. The BC-SPIs of the present invention contained 50% BC, compared to 25% in commercial SPI as measured by SDS-PAGE gel electrophoresis methods defined in Example 1. Also the BC-SPI of the present invention contained approximately 7% glycinin compared to 51% in commercial SPI.

### Solubility and color of BC-SPI

High BC soybeans were harvested near the St. Louis area in Illinois in the fall of 1997. The seeds were processed into BC-SPI by using acid precipitation and a combination of ultrafiltration and diafiltration methods (Lawhon, J., U.S. Patent #4,420,425, 1983; Koseoglu, S. and Lusas, E., In "Proceedings of the World Congress on Vegetable Protein Utilization in Human Foods and Animal Feedstuffs, e.g. Applewhite, T., Amer. Oil Chem. Soc., Champaign, IL, pp. 528-547, 1989) as described in Example 3. Low-BC seeds (BC is 11 % of total protein) were also processed into soy protein isolate for comparison in animal feeding studies. In one embodiment of the invention, the solubility and color of the BC-SPI made by ultrafiltration and diafiltration was substantially better than the commercial isolates and BC-SPI made by acid precipitation as discussed in Example 4. This finding is valuable for dry beverage mix applications where only low shear mixing is used to disperse the powder and where the white color of dairy beverages is desired. Insoluble aggregates larger than 10 microns are detected by the consumer as an objectionable gritty mouthfeel. A much smaller portion of the particle volume in the hydrated BC-SPI made by ultrafiltratration and diafiltration came from particles greater than 10 microns as described in Example 3.

### Stability of BC-SPI near pH 6.7. beverage model

The properties of the BC-SPIs were compared to commercial soy protein isolates and sodium caseinate in a model beverage system, pH 6.7. The BC-SPIs performed as well or better than commercial isolates depending on the conditions of pH for example as shown in Example 5. It was discovered that BC had greater stability against calcium ion induced aggregation than commercial SPI, but not the same degree of calcium stability that we discovered for purified BC as shown in Example 2. This invention also includes the discovery that the BC-SPIs of the present invention have a high degree of freeze-thaw stability which was better than that of whole and partially-hydrolyzed commercial SPI as discussed in Example 5. The freeze-thaw stability of BC-SPIs is lost when free calcium ions are included in the formation. Therefore an aspect of the invention is the development of frozen foods containing little free calcium ions, and using BC-SPI to achieve good freeze-thaw stability.

### Thickening properties of BC-SPI at pH 5.8, an emulsified meat model

Commercial soy protein isolates are typically heat-treated at or above 90 degrees C for up to 20 seconds during manufacture to denature the proteins and form a complex between the basic subunits of glycinin and subunits of beta-conglycinin. This facilitates the gelation of the soy protein isolates in food products, especially at temperatures which fall below the denaturation temperature of the proteins. To test the application of BC-SPI for gelling properties at the pH, salt and temperature conditions of emulsified meats, we compared the gelling properties of BC-SPI which was preheat-treated (90 degrees C) with the gelling properties of commercial isolate and egg white. One unexpected finding of the present invention was that the viscosities of the emulsions prepared with BC-SPIs were 1.7-3.0 times greater than those prepared with the commercial soy protein isolate as shown in Example 6. Furthermore the water-holding structures formed by protein-protein interactions between the surfaces of protein-coated fat droplets were more easily broken down in the emulsions stabilized by BC-SPI than in those stabilized by commercial isolate, as measured by the greater decreases in viscosity with increase in shear time as discussed in Example 6. When this breakdown occurs in the mouth it is perceived as a more desirable texture, e.g., smoother and less tough. Therefore, the gelling (or fat and water binding) properties of soy protein isolate under the conditions of emulsified meat systems can be optimized by using BC-SPI.

### Amino acid composition of BC soybeans and BC-SPI

High BC soybeans of this invention were surprisingly high in protein and in the contents of methionine, cysteine, lysine and arginine amino acids as indicated in Example 6. These amino acids are normally limiting in soybeans and soybean meal, especially for infant animals and humans (DeLumer, B. et al., Food Technol. 51:67-70, 1997). Therefore one aspect of the present invention includes the use of high BC soybeans to make soybean meal (full fat and defatted) which is rich in essential amino acids for use in animal feed, limiting the amount of synthetic amino acids that are needed to fortify feed rations.

The beta-conglycinin rich soy protein isolates made by the processes described in Example 3, were either similar in essential amino acid composition to that of commercial soy protein isolates or rich in sulfur amino acids as shown in Example 8. So the uses of the BC-SPI for various food texture and physiological benefits are not necessarily limited by an imbalance of essential amino acids. The likely explanation is that the high BC soybeans were also enriched in minor soy proteins as part of the compensation for the loss of glycinin and these proteins are retained in the soy protein isolate.

### Cholesterol-lowering properties of BC-SPIs

Soybeans which lack the alpha and alpha-prime subunits of BC and BC rich soybeans were used to make soy protein isolates. These BC-SPIs and control isolates made from normal soybeans are being tested for cholesterol lowering properties using hamsters and published methods (Terpstra, A., et al., J. Nutr. 121:944-947, 1991; Potter, S., et al., J. Nutr. 126:2007-2011, 1996; Balmir, F. et al., J. Nutr. 126:3046-3053, 1996). According to the invention, the BC produced has several beneficial effects. In particular, BC has specific cholesterol-lowering benefits related to the cholesterol-lowering properties of soy protein ingredients and foods. These benefits are related to the BC contents not the total soy protein contents.

### Low cysteine BC-SPI for processed cheese applications

In cheese applications of the present invention, protease-treated BC-SPI functions more similar to casein proteins than commercial SPIs. There are several differences between the structure of casein proteins and soy proteins. One key difference is that the primary caseins (alpha-s1- and beta-caseins) lack cysteine, whereas the five soy glycinin subunits each contain 6-8 cysteines and the beta-conglycinin subunits contain 0 or 1 cysteine (Utsumi et al. In Food Proteins and Their Applications, Eds. Damodaran, S. and Paraf, A., Marcel Dekker, Inc., 1997). Beta-conglycinin is more similar to caseins in this respect than glycinin, so fewer defects caused by sulfur groups are likely in the cheese lacking glycinin (e.g. sulfur-based flavors, disulfide-linked protein aggregates which hold water and create mealy texture).

Another key difference between casein proteins and soy proteins is that the caseins are phosphorylated. The phosphate groups of casein proteins serve two roles. The first role is to make the casein proteins insoluble in the presence of calcium following the addition of the enzyme chymosin which specifically cleaves kappa-casein. The second role is to resolubilize or hydrate the caseins following acidification by bacterial cultures, and removal of water (whey). At the lower pH, calcium is released from the phosphoproteins and the hydrated phosphate groups limit protein-protein interactions. In processed cheese, the pH does not have to be lowered as far to solubilize the caseins, because various phosphate salts are added which chelate calcium ions. The limited nature of the casein-casein interactions at pH of 5.1-6.0 and at food preparation temperatures, reduces the water-binding and hence viscosity of the protein-stabilized oil-in-water emulsion, and enables the caseins to flow as demonstrated by the melting and stretching properties of cheese when it is heated. The solubilized caseins also serve a critical role as emulsifying agents in processed cheese, which prevent the separation of fat during cooking.

Beta-conglycinin is a glycoprotein which contains covalently attached carbohydrates. These carbohydrates which remain hydrated at the pH of cheese, limit protein-protein interactions between BC proteins. Another way of improving the solubility of BC proteins at the pH of cheese is to partially hydrolyze BC. Modification of normal SPI improved the melting property of cheese analogs (Kim, S. et al., JAOCS 69:755-759). Further improvement is made by using GC or glycinin rich hydrolysates. In the instant invention the partial hydrolysis of BC is uniquely valuable because the BC proteins are especially susceptible to being cleaved in half to form 30 kDa fragments (Kawai et al., Biosci. Biotech. Biochem. 61:794-799, 1997). An aspect of the invention is the discovery that partially hydrolyzing BC-SPI works well in processed cheese analogs as discussed in Example 9.

The processed cheese making properties of BC are improved by partially hydrolyzing BC using an enzyme such as Alacalase (Novo Nordisk) as shown in Example 9. The removal of cysteine-rich proteins from BC-SPI will also improve the properties of hydrolyzed BC in processed cheese.

### Low methionine, high arginine protein for homocysteine

Methionine derived from dietary proteins serve as one of the major sources for the biosynthesis of sulfur amino acids including homocysteine. Homocysteine is a key risk factor of cardiovascular disease. Prolonged consumption of low methionine diet will, therefore, reduce or at least maintain the plasma level of homocysteine.

Arginine, on the other hand, is the natural substrate for nitric oxide synthase, which converts arginine to citrullin and nitric oxide (NO). Endothelial cytotoxicity of homocysteine is modulated by the presence of NO; NO and homocysteine react under physiological conditions to form the non-toxic S-nitroso-homocysteine. Furthermore, NO is an important vascular mediator, released continuously by endothelial cells in the basal state. In the hypercholesterolemic rabbit model, dietary supplementation with L-arginine reduces atheroma formation, improves endothelium-dependent dilation, decreases platelet aggregation and monocyte adherence to aortic endothelium. It has also been shown that L-arginine supplementation inhibits platelet aggregation in healthy young adults via the nitric oxide pathway. In hypercholesterolemic humans, L-arginine supplementation has been shown to improve endothelium-dependent vasorelaxation. Furthermore, nitric oxide, which is known to serve as a negative feedback for VEGF, has an added benefit in preventing angiogenesis and metastasis. Thus, with those known health benefits of continuously released low levels of nitric oxide, in the present invention a high arginine and low methionine containing soy protein is used as a natural approach to detoxify homocysteine and represents a viable and attractive alternative to current therapy of using vitamin supplements. The preparation of this aspect of the invention is discussed in Example 10.

### Low sulfur amino acid protein ingredient to reduce the risks of cancer and osteoporosis.

Methionine plays a critical metabolic role in tumor development by ultimately promoting protein synthesis and cell proliferation. Thus, the lower methionine content of soy proteins compared to animal proteins such as casein contributes to the inhibition of tumorigenesis by low methionine proteins such as commercial soy protein isolate (Hawrylewicz, E. and Huang, H., In "Dietary Proteins, How They Alleviate Disease and Promote Better Health", Liepa, G., editor, Amer. Oil Chem. Soc., Champaign, IL, pp. 123-150, 1992). The use of an even lower methionine containing BC-SPI in high protein food applications will further improve the safety of consuming high protein foods.

The composition of proteins in our diet also influence bone health by influencing our retention of dietary calcium. Higher urinary calcium excretion for subjects on an animal protein diet is related to higher content of sulfur containing amino acids (Breslau, N. et al., J. Clin. Endocrinol. Metab. 66:140-146, 1988) and is related to higher incidence of hip fractures in women who have higher animal intakes (Abelow, B. et al., Calcif. Tissue Int. 50:14-18, 1992). One of the mechanisms involved is as follows: sulfur is oxidized to sulfate in vivo, which generates a fixed acid load that is buffered by bone, resulting in bone dissolution. The low content of sulfur amino acids of low methionine BC-SPI is beneficial in the prevention of osteoporosis, cancer and heart disease.

### Functional food applications

The above physiological benefits of beta-conglycinin for functional food applications have not been recognized by food product developers. Using the new soybean varieties of the present invention, it is now possible to make BC-SPIs which are used to make beverages and cheese analogs with improved texture, flavor, color and nutritional quality.

### Additional modifications

Also included are additional modifications of beta-conglycinin-rich soybeans and beta-conglycinin proteins which further extend the efficiency of isolate manufacture and the usefulness of BC-SPI. Examples include the following: 1) Reduction of the amount of non-storage proteins in the soybean to increase the yields of beta-conglycinin; 2) addition of single, double or triple-lipoxygenase-null trait to reduce off-flavor development during BC-SPI manufacture as discussed in Example 11; 3) reduction in the content of linoleic and linolenic acid in the soybean, for example by increasing oleic and or stearic acid, to reduce off-flavor development during BC-SPI manufacture; 4) modify the amounts of alpha, alpha' and beta-subunits of beta-conglycinin to obtain various benefits (e.g. reduce allergenicity, improve solubility) by using antisense technology or the compositions of the subunits by using site-specific mutagenesis; 5) partial enzymatic hydrolysis of the beta-conglycinin-rich isolate to improve protein solubility, cheese analog properties, gelling and foaming properties; and 6) enzymatic phosphorylation or deamidation of beta-conglycinin proteins to improve solubility and related functional properties.

Additional procedures and methods are well known in the art. Suitable procedures, materials and methods may be found in Fox, P.F., Cheese: Chemistry, Physics and Microbiology, Vol. 2, Major Cheese Groups, pp 339-383, (1987); Kolar, C.W., et al., J. Amer. Oil chem. Soc. 56:389-391, (1979); Fukushima, D., Food Rev. Int. 7:323-351, (1991); Hoogenkamp, H.W., Vegetable Protein Technology Value in Meat, Poultry & Vegetarian Foods, (1992); Mead, G.C. 1989, Processing of Poultry, Elsevier Appl. Sci., NY; Forrest, J.C. et al. 1975, Principles of Meat Science, W.H. Freeman Co., San Francisco; and Wilson, L.A., Amer. Oil Chem. Soc., (1995),

### EXAMPLES

The following examples are provided to further illustrate the present invention. Examples not covered by the scope of the claims were kept for illustrative purposes.

### Example 1

### SDS-PAGE gel electrophoresis of soybeans and soy protein isolate

1) Weighed out 5mg of sample of known protein content determined by kjeldahl, (protein = nitrogen x 6.25) and placed it in a 650 microliter microcentrifuge tube.
2) Added 500 microliters of SDS sample solubilizing solution (containing 10 % glycerol, 2.3 % SDS, 0.0626 M Tris pH 6.8, 5 % 2-mercaptoethanol, 0.05 % Bromophenol Blue).
3) Seal tubes and in the case of SPI samples placed in shaker for 20 minutes. Placed in boiling water bath for 10 minutes.
4) Cooled samples to room temperature.
5) Spun samples in microcentrifuge (~14,000 x g) for 10 minutes.
6) Recovered supernatant.
7) Loaded 5-8 microliters (20-30 micrograms protein).

The running/staining conditions:
1) All gels had been analyst-cast 10-20 % total acrylamide (T), and 2.67 % Laemelli gels that were 0.75 mm thick. The gels were cast and run using the Bio-Rad Protean II xi system which creates gels which are approximately 16cm x 16 cm.
2) Stacking gels were cast with 15 wells, and samples were not run in the outer lanes. Bio-Rad Broad Molecular weight standards were loaded on each side of the gel to be used for apparent molecular weight determination.
3) The gels were run using constant voltage (60-100 volts overnight) or constant current (15-30 mA/gel for 6-8 hours).
4) Gels were fixed in 12.5 % trichloroacetic acid for at least 1 hour at room temperature.
5) The gels were removed from TCA and rinsed briefly in deionized water. They were placed in colloidal Coomassie Solution A (11 % Ammonium sulfate/ 2 % Phosphoric acid) for 1 hour at room temperature, then placed in staining solution composed of 160 ml of solution A, 40 ml of methanol, and 4.6 ml of solution B (1 gm Coomassie G250 in 20 ml of deionized water).
6) The gels were stained at room temperature in this solution for at least 16 hours (>24 is best), then they were rinsed with deionized water and placed in 7 % acetic acid to wash away the trace of background staining that existed. At this point gels were ready for imaging or photography.

Digital images of Coomassie stained gels were generated using a Kodak Videk Mega-plus charge-coupled device (CCD) array camera and scanning software included with a Biolmage Visage 2000 Image analysis system. The CCD array camera generated digital images which are 1024 x 1024 pixels, with 256 digital values representing a range of optical density. During the image acquisition process, the system was calibrated with regard to pixel size and digital values. The gels were scanned using transmitted light, a 17 mm lens, 2 neutral density filters, and a yellow filter to enhance contrast of the Coomassie stain.

Analysis of the digital images was performed using Biolmage Whole Band analysis software. With this software, the analyst provided lane boundaries, and the software identified bands then generated their boundaries on an automated basis. The analyst had the ability to remove band boundaries which failed to represent the band, and the ability to assist in the determination of boundary placement by indicating the main axes of the band. This method controls analyst bias by applying the same algorithms to the boundary determination process. It was also possible for the analyst to manually define (draw) the band boundary, but this approach was only used where all other approaches failed (typically less than 2% of the bands). The whole band software quantitated individual bands by summing the digital values for each pixel within the band boundary, generating a value known as integrated optical density or IOD. Commercial molecular weight standards (Bio-Rad Broad Molecular Weight Standards Catalog #161-0317) run in individual lanes on the same gel are used to determine the apparent molecular weight of bands in samples. Individual bands may be named by the analyst to aid in their identification on the bandlist.

The results of image analysis were presented in tabular form as a "bandlist". The bandlist contains data grouped by lanes including the band number (numbered from the top of the lane to the bottom), band name, band IOD, % IOD (% of all quantitated material in the lane represented by this band), and molecular weight. Copies of the computer screen were printed as "screendumps". Screendumps were generated showing the digital gel image with no annotation in place, with centers of quantitated bands indicated by a dashed line, and with full annotation showing land boundaries, lane names, band centers, band boundaries, and molecular weight standards.

### Example 2

Commercial soy protein isolates, Supro 760 (Com'l SPI A) and Supro 940 (Com'l SPI B), were obtained from Protein Technologies International, St. Louis Missouri. Commercial soy protein concentrate, Promine DS (Com'l SPC), was obtained from Central Soya company, Inc., Fort Wayne, Indiana. Whole protein isolate (Lab. SPI) was prepared according to the method of Boatright, W.L., et al., J. Amer. Oil Chem. Soc. 72:1439-1444, (1995), except petroleum ether was used to extract the fat. Emulsions containing protein (1% protein), peanut oil (10%), sucrose (5% in water phase), NaCl (70 mM, 0.4% in water phase), and CaCl2 where indicated (4 mM) were prepared by sonication {160 watts, 60 sec.) and median particle diameter measurements were determined using a Malvern mastersizer laser light diffraction instrument. A Bohlin VOR rheometer was used to determine the viscosity of heat-treated samples (14.6 1/sec. shear rate, 5 min., 5 degrees C). The emulsions were frozen (4 days, -14 degrees C) and thawed or heat-treated (20 mL in glass vial submerged in 90 degrees C water bath for 60 minutes).

The small particle diameters of the heat-treated or frozen-thawed emulsions prepared with sodium caseinate and beta-conglycinin demonstrate the potential of beta-conglycinin for replacing sodium caseinate in emulsion applications near pH 6.7, such as nutritional beverages and coffee creamers as shown in Table 1.

**Table 1**

| **Median particle diameters of emulsions stabilized by purified beta-conglycinin, laboratory soy protein isolate and commercial protein ingredients** | | | | |
|---|---|---|---|---|
| Protein | 0.4% NaCl | | 0.4% NaCl, 4 mM CaCl2 | |
| | pH 6.0-6.1 | pH 6.6-6.8 | pH 6.0-6.1 | pH 6.6-6.8 |
| Sodium Caseinate | 1.0 | - | 1.1 | 1.1 |
| Beta-conglycinin | 1.2 | 1.2 | 9.8 | 3.4 |
| Lab. SPI | 14.2 | 2.2 | 36.3 | 14.1 |
| Com'l SPI A | 30.0 | 1.6 | 69.1 | 61.9 |
| Com'l SPI B | 82.7 | 56.0 | 89.2 | 81.8 |
| Com'l SPC | 46.6 | 44.6 | 49.7 | 60.4 |

**Table 2**

| **Median particle diameter or heat-treated and freeze-thawed emulsions stabilized by purified beta-conglycinin, laboratory soy protein isolate and commercial protein ingredients, 0.4% NaCl, 5% sucrose.** | | | | |
|---|---|---|---|---|
| Protein | Heat-treated | | Freeze-thawed | |
| | pH 6.0-6.1 | pH 6.6-6.8 | pH 6.0-6.1 | pH 6.6-6.8 |
| Sodium Caseinate | 1.0 | - | 1.0 | - |
| Beta-conglycinin | 3.1 | 1.4 | 4.0 | 13.8 |
| Lab. SPI | 39.1 | 11.5 | 55.0 | 88.0 |
| Com'l SPI A | 34.9 | 1.9 | 83.9 | 75.3 |
| Com'l SPI B | 72.4 | 49.9 | 111 | 104 |
| Com'l SPC | 47.7 | 49.9 | 111 | 98.8 |

**Table 3**

| **Viscosity of heat treated emulsions stabilized by purified beta-conglycinin, laboratory soy protein isolate and commercial protein ingredients, 0.4% NaCl, 5% sucrose.** | | | | | |
|---|---|---|---|---|---|
| Protein | | | | | |
| pH | Beta-conglycinin | Lab. SPI | Com'l SPI A | Com'l SPI B | Com'l SPC |
| s | mPa s | mPa s | mPa s | mPa s | mPa s |
| 6.6 | 50 | 180 | 60 | 70 | 230 |
| 6.1 | 60 | 650 | 270 | 50 | 140 |
| 5.6 | 1,490 | 270 | 110 | 30 | 110 |
| 5.0 | 120 | 30 | 70 | - | 100 |

### Example 3

### Pilot plant preparation of high beta-conglycinin soy protein isolates

### A. Removal of fat from soybeans

1. Adjust soybeans to about 10% moisture and temper at room temperature.
2. Crack soybeans by using a cracking mill.
3. Dehull the cracked soybeans by using a Kice Aspirator.
4. Condition the cracked and dehulled soybeans at 50-60 degrees C by using a cooker.
5. Flake the conditioned soybeans using a Flaking Mill.
6. Extract soybean flakes with hexane.
7. Desolventize the defatted soybean meal in a laboratory fume hood for three days.

### B. Acid precipitation

1. Water was added to a 200 liter jacked tank and adjusted to 27-35 degrees C and pH 8.5-9.0 using 20% NaOH. Defatted soybean flakes were added and mixed with an agitator equipped with two propellers and a powered in-line 3-stage mixer which was fitted with fine, medium and coarse rotors. The powered in-line mixer with a closed loop circulation was used throughout the extraction process to reduce the particle size of the flakes and improve protein extraction. The water to soy flour ratio was 10/1 (w/w). The pH of the slurry was readjusted to pH 8.5-9.0 if pH of the slurry is below 8.5.
2. The solubilized soy protein was recovered from the extraction slurry by centrifuging (25-35 degrees C), first with a decanter to remove the majority of the spent solids, followed by clarification of protein-containing liquid in a desludging disk centrifuge at a feed rate of 250-500 kg/h.
3. The pH of the clarified protein solution was adjusted to 6.8-7.0 using 6% hydrochloric acid. The protein solution was pasteurized at a low heat condition (e.g. 72 degrees C for 15 seconds) or a high heat condition (e.g., 90 degrees C for 20 seconds) depending on the application, using a plate and frame heat exchanger and cooled to 25-35 degrees C.
4. The pasteurized protein solution was adjusted to pH 4.5 +/- 0.1 by adding hydrochloric acid (12%) and allowed to react for 30 minutes at 30-35 degrees C.
5. The precipitated protein was recovered using a desludging disk centrifuge at a feed rate of 200-400 kg/h and desludging interval of 3 minutes.
6. The protein curd was washed for 10-30 minutes, two times using acidified water (pH 4.5 +/- 0.1, 30-35 degrees C). Ratio of washing water to packed wet solids was 5:1 (w/w). Any lumps in the curd were broken up using a powered in-line mixer. The protein curd was recovered using a desludging disk centrifuge at a feed rate of 350-450 kg/h after each washing.
7. The washed curd was mixed with dilute sodium hydroxide (0.5%) to neutralize (pH 7.0-7.2), diluted with water to 12-15% solids (preferably 15% solids) and readjusted to pH 7.0-7.2. A powered in-line mixer homogenizes the slurry before spray-drying. The protein solution was stored at 4 degrees C or as cool as possible before spray drying using a heat exchanger.
8. The neutralized and homogenized protein solution was adjusted to 45-55 degrees C and was spray dried using an inlet air temperature of 180-185 degrees C, outlet air temperature of 85-90 degrees C.

### C. Ultrafiltration and diafiltration

1. Pasteurized protein was prepared and solution from step A-3 above was obtained.
2. The protein solution was passed over an ultrafiltration membrane (e.g. hollow fiber) with a molecular weight cutoff of 100,000 Daltons. The original volume of the protein solution was maintained in a feed container during ultrafiltration and diafiltration by adding water to make up for the removed permeate.
3. The redounded solution was recycled to the feed container.
4. Once the permeate was about 1.3 to 1.5 times the original feed volume, the addition of water to the feed container was discontinued and the permeate was collected (e.g. 90 degrees C - 120 degrees C for 20 seconds).
5. The volume of the feed container was reduced by ultrafiltration until a solids content of about 15% solids was achieved and adjusted to pH 6.8-7.0 by adding 8% NaOH or 6% HCl.
6. The redounded was spray dried using an inlet air temperature of 180-185 degrees C and outlet air temperature of 85-90 degrees C.

### Example 4

### Table 4. Nitrogen solubility index and color of the BC-SPI made by ultrafiltration and diafiltration compared to commercial isolates

BC-SPI isolates and low-BC isolate (for animal feeding study) were made by the acid precipitation and ultrafiltration methods at POS Pilot Plant Corp., Saskatoon, SK, Canada according to the methods in Example 3. Compositions and physical properties of the isolates compared are presented in the tables below. There is twice as much BC in BC-SPI compared to Com'l SPI A, using gel electrophoresis methods of Example 1. The main glycinin component of the BC-SPIs is the A3 submit (3.6% of the proteins in the soybean). The low BC SPI are lacking the alpha and alpha' subunits of beta-conglycinin. Since both low BC SPI and (high) BC-SPIs have similar amino acid compositions as indicated in Example 8 and isoflavone content, any differences that we see in the cholesterol lowering properties of the isolates reflect different qualities of soy protein.

| Protein isolates | NSI | Hunter colorimeter values | | P. Vol>10 m.* Med.** | |
|---|---|---|---|---|---|
| | % | L (whiteness), | b (yellowness) | % | Microns |
| BC-SPI (UF) | 96 | 89.3 | 8.34 | 22.5 | 0.33 |
| BC-SPI (acid) | 34 | 85.5 | 11.8 | 54.2 | 22.2 |
| Com'l SPI A | 63 | 85.5 | 15.4 | 74.4 | 65.8 |
| Com'l SPI C | 82 | 82.8 | 13.5 | 0 | 0.38 |

| | | | | | |
|---|---|---|---|---|---|
| P.H. = partially hydrolyzed *P. Vol> 10 m. - particle volume from particles greater than 10 microns **Median particle diameter | | | | | |

**Table 4. Chemical composition of SPIs**

| **Component as is %** | **BC-SPI (Acid) (%)** | **BC-SPI (UF) (%)** | **Low-BC SPI (Acid) (%)** | **Com'l SPI A (%)** |
|---|---|---|---|---|
| Protein | 87.4 | 87.5 | 89.7 | 86.8 |
| Moisture | 4.89 | 5.21 | 4.97 | 6.21 |
| Fat (acid hydrolysis) | 4.35 | 3.01 | 3.82 | - |
| Ash | 3.67 | 3.46 | 3.7 | |
| Beta-conglycinin | 49.0 | 50.1 | 12.4 | 25.1 |
| Glycinin | 7.1 | 7.0 | 55.2 | 51.1 |

**Table 5. Isoflavone content in SPIs. The isoflavones values listed are the sums of the individual isomers of each isoflavone, genistein, daidzein and glycitein, normalized for their molecular weight differences to give the total isoflavone concentrations**

| **Component** | **BC-SPI (Acid) (micrograms/g sample dry weight)** | **Low-BC SPI (Acid) (micrograms/g sample dry weight)** |
|---|---|---|
| Genistein | 594 | 418 |
| Daidzein | 188 | 148 |
| Glycitin | 45 | 73 |
| Total isoflavones | 827 | 639 |

### Example 5

### Properties of BC-SPIs compared to commercial SPIs and sodium caseinate in a model beverage system

**Materials:** Commercial SPI (Com'l SPI A) and partially hydrolyzed commercial soy protein isolate (Com'l SPI C) were obtained from Protein Technologies International, St. Louis, Missouri.

**Emulsion formation:** Protein (final concentration of 1%, using 5.71 x nitrogen for soy proteins and 6.38 x nitrogen for casein) (Morr, C.J., Food Sci. 47:1751, 1982) was slowly added to a 5% sucrose solution using a Dispermat mixer and 0.4% NaCl (in water phase) was added to the mixture. The initial pH of the solutions were adjusted according to the previous experiments to that the final pH of the solution will be close to the targeted pH. Peanut oil was slowly added to the protein solution in the Dispermat (about 3 minutes). The formulations were sonicated (160 watts) for 1 minute in the 50ml plastic beaker with sonication probe at a depth of the 20ml mark in the beaker.

**Heat-treatment:** Protein-stabilized emulsions (20 mL) were transferred to glass vials with screw top lids, submerged in 90 degrees C water bath for 60 minutes and stored in a refrigerator overnight.

**Particle size and viscosity measurements:** Samples were tested for viscosity using a Bohlin rheometer (C14 cup and serrated bob, 5 minute equilibrium time, 5 degrees C, 14.6 1/second shear rate, 5 minutes of shear), and were tested for median particle diameter using a Horiba LA910 particle size analyzer (volume basis, relative refractive index of 1.10, lowest circulation speed.

**Results:** Sodium caseinate is a good emulsifier and a stable protein against aggregation as illustrated by the small median particle diameters (0.9-1.0 microns) of the emulsions prepared under various conditions. The casein-stabilized emulsion at pH 6.7 in the presence of 4 mM CaCl₂ did not show any separation of serum during storage. Unlike Com'l SPI A, both BC-SPIs exhibited good freeze-thaw stability as indicated by the small median particle diameters of the emulsion droplets (3.5 +/- 0.2 microns). The BC-SPI (acid) stabilized emulsion droplets showed the best stability against aggregation, of the soy protein samples, at pH 6.5, as indicated by the small median particle diameter (2.9 microns). However, the BC-SPI UF stabilized emulsion droplets were most stable of the soy protein samples, against aggregation in the presence of calcium ions (pH 6.7).

**Table 6. Median particle diameters of protein-stabilized emulsions prepared or stored under pH, salt and freezing conditions indicated. All emulsions contained 0.4% NaCl and 5% sucrose in the water phase and 1% protein, 10% peanut oil.**

| Protein | pH 6.7 | pH 6.5 | pH 6.7, Freeze-thaw | pH 6.7, 4 mM Ca | Free serum after 1 day 4 mM Ca |
|---|---|---|---|---|---|
| | (microns) | (microns) | (microns) | (microns) | (mL) |
| Casein | 0.9 (0.02) | 1.0 (0.003) | 0.9 (0.03) | 0.92 (0.0) | 0 |
| BC-SPI (UF) | 1.3 (0.2) | 4.9 (0.1) | 3.3 (1.1) | 10.6 (0.4) | 1.5 |
| BC-SPI (acid) | 1.7 (0.3) | 2.9 (0.2) | 3.7 (0.2) | 19.8 (0.4) | 5.0 |
| Com. SPI | 1.8 (0.4) | 7.7 (0.3) | 86.9 (1.8) | 36.7 (0.4) | 10.0 |
| Com. SPI-P.H. | 13.1 (1.9) | 13.8 (0.8) | 10.8 (0.14) | 11.6 (0.1) | 0.5 |
| Parentheses show standard deviation of duplicate samples. | | | | | |

**TABLE 7. Median particle diameters of protein-stabilized emulsions which were heat-treated (90 degrees C water bath for 30 minutes).**

| | | | | |
|---|---|---|---|---|
| BC-SPI (acid) had the same degree of heat-stability as commercial SPI at pH 6.7 and had better heat-stability at pH 6.5 than both commercial SPI and BC-SPI made by ultrafiltration. | | | | |

| **Protein** | **pH 6.7 (microns)** | | **pH 6.5 (microns)** | |
|---|---|---|---|---|
| Casein | 0.9 | (0.01) | 0.9 | (0.01 |
| BC-SPI (UF) | 5.1 | (1.3) | 10.6 | (0.7) |
| BC-SPI (acid) | 4.6 | (1.2) | 6.5 | (1.4) |
| Com. SPI | 4.3 | (0.6) | 9.7 | (0.02) |
| Com. SPI-P.H. | 14.2 | (1.6) | 13.8 | (3.0) |

Parentheses show standard deviation of duplicate samples.

### Example 6

### Texturizing properties of BC-SPIs under conditions modeling an emulsified meat system

**Materials:** Commercial soy protein isolate (Com'l SPI A) is manufactured as a high heat SPI. BC-SPI samples were made by the methods in Example 2. High heat BC-SPI can also be made by dispersing low-heat BC-SPI in an aqueous solution in a glass vial and submerging the vial in a 90 degrees C water bath for 30 minutes. Commercial soy protein isolate was from Protein Technologies International, St. Louis Missouri. Egg white was from Canadian Inovatech Inc., Abbotsford, British Columbia.

**Emulsion formation:** Protein (final concentration of 1%, using 5.71 x nitrogen for soy proteins and 6.25 x nitrogen for egg white protein) was slowly added to a 5% sucrose solution using a Dispermat mixer and 3.5% NaCl (in water phase) was added to the mixture. The initial pH of the solutions were adjusted according to the previous experiments so that the final pH of the solution will be close to the targeted pH. Peanut oil was slowly added to the protein solution in the Dispermat (about 3 minute). The formulations were sonicated (160 watts) for 1 minute in the 50ml plastic beaker with sonication probe at a depth of the 20ml mark in the beaker.

**Gelation:** 20ml of the above each sample was heated in a glass vial in a 70 degrees C water bath for 30 minutes or in an 80 degrees C water bath for 30 minutes, then stored in a refrigerator (5 degrees C) over night.

**Particle size and viscosity measurements:** Samples were tested for viscosity using a Bohlin rheometer (C14 cup and serrated bob, 5 minute equilibrium time, 5 degrees C, 14.6 1/sec. shear rate, 5 min. of shear), and were tested for median particle diameter using a Horiba LA91 0 particle size analyzer (volume basis, relative refractive index of 1.10, lowest circulation speed).

**Results:** Egg white is an excellent gelling agent used in numerous foods including meat products such as surimi (crab leg analogs). However, like meat protein, egg white is expensive compared to soy protein. The soy protein ingredients which came closest to performing like egg white, under conditions which mimic an emulsified meat system, were the high heat BC-SPIs as shown in Table 8. The manufacture of high heat BC-SPI is done at neutral pH where protein denaturation occurs but aggregation of the denatured proteins is inhibited. The highest viscosities or firmness or water and fat binding occurs when denatured protein is exposed to conditions which are optimum for the formation of fine gel/aggregate structures. The gelling condition is near pH 5.8 for BC and gelation of BC can occur at or below the denaturation temperature of BC when BC is predenatured. Low-heat BC-SPIs were also tested for comparison. The difference between the viscosities of the samples made with low-heat BC-SPIs and those made with high-heat BC-SPI quantify the value added by predenaturing the soy proteins using a high heat treatment. There is less value added by preheating normal SPI because glycinin forms complexes with BC changing the nature of subsequent gelling reactions (e.g., optimum gelling conditions are moved to different pH values and there are fewer non-aggregated sites on BC available for gel formation).

**Table 8. Viscosities (in units of mPa s) of the heat-treated emulsions prepared with BC-SPIs compared to those prepared with commercial soy protein isolate, pH 5.8, 3.5% NaCl in water, 5% sucrose in water.**

| | Viscosity | Viscosity | Viscosity | Viscosity |
|---|---|---|---|---|
| | (70°C) | (70° C) | (80° C) | (80°C) |
| Protein isolate | 5 sec shear | 5 min. shear | 5 sec. shear | 5 min. shear |
| Egg white | 464 (17) | 327 (14) | 1800 (375) | 1157 (287) |
| High heat BC-SPI (UF) | 222 (21) | 189 (13) | 341 (10) | 278 (3) |
| High heat BC-SPI (acid) | 229 (17) | 199 (6) | 404 (32) | 317 (9) |
| Low heat BC-SPI (UF) | 149 | 138 | 229 | 202 |
| Low heat BC-SPI (acid) | 142 | 126 | 193 | 167 |
| "High heat" Com'l SPI A* | 74 (4) | 73 (4) | 149 (25) | 145 (6) |
| Com'l SPI A | 115 (0) | 111 (3) | 185 (4) | 164 (3) |

| | | | | |
|---|---|---|---|---|
| *Commercial SPI dispersion in a glass vial was heated in a 90 degrees C water bath for 30 minutes and cooled in a refrigerator. "High heat" is in quotes because commercial SPI is highly heat-treated during commercial manufacture. This test shows that further heat-treatment does not improve the isolates performance. Figures in parentheses indicate standard deviation values for duplicate tests. | | | | |

### Example 7

Protein and amino acid compositions of BC soybeans were compared to average composition of 58 diverse lines of soybeans as indicated in Tables 9 and 10. Soybeans (7-10 grams) were finely ground and analyzed for amino acid composition, protein and moisture by Ralston Analytical Laboratories, St. Louis, Missouri according to standard procedures. The beta-conglycinin rich soybeans had high levels of protein, cysteine, methionine, arginine, and lysine which are especially valued for animal feed.

**Table 9. Showing amino acid and protein content in units of: percent, dry basis. The elite line is Hartz 5350.**

| Amino acid | Avg. of 58 lines | Range | Elite line | High BC |
|---|---|---|---|---|
| Lysine | 2.42 | 2.11-2.66 | 2.65 | 2.91 |
| Methionine | 0.49 | 0.41-0.57 | 0.55 | 0.62 |
| Cysteine + Met | 1.03 | 0.84-1.19 | 1.14 | 1.44 |
| Threonine | 1.43 | 1.35-1.59 | 1.52 | 1.54 |
| Tryptophan | 0.48 | 0.42-0.55 | 0.50 | 0.48 |
| Arginine | 2.74 | 2.36-3.51 | 2.91 | 3.40 |
| Total protein | 41.0 | 37.4-44.8 | 41.0 | 43.0 |

**Table 10. Amino acid data for soybeans in units of mg/gram protein:**

| Amino acid | Avg. of 58 lines | Range | Elite line | High BC lines |
|---|---|---|---|---|
| Lysine | 59.6 | 49-66 | 64.6 | 68.0 |
| Methionine | 12.0 | 10.5-13.4 | 13.5 | 14.5 |
| Cysteine + Met | 25.3 | 21-29 | 28.0 | 33.8 |
| Threonine | 35.2 | 30-40 | 37.2 | 36.1 |
| Tryptophan | 11.8 | 10.8-13.0 | 12.1 | 11.1 |
| Arginine | 67.4 | 57-78 | 71.0 | 79.5 |

### Example 8

### Amino acid composition of soy protein isolates

**Table 11**

| Amino Acids | Low BC (mg/g protein) | Hi BC (mg/g protein) | UF-Hi BC (mg/g protein) | Com'l SPI-A | FAO.WHO Requirements 2-5 yrs old |
|---|---|---|---|---|---|
| Cystine (Performic) (G/100G sample) | | | | | |
| Cysteine | 12.49 | 12.24 | 18.51 | 11.52 | |
| Methionine | 14.16 | 13.04 | 14.40 | 12.56 | |
| Total sulfur amino acids | 26.64 | 25.29 | 32.91 | 24.08 | 25 |
| Alk Hydrol. Trypt. (G/110G) | 14.27 | 11.21 | 10.51 | 11.18 | 11 |
| | | | | | |
| Amino Acids (Hi Vac) | | | | | |
| Units=GM/100 GM "As Is" Basis | | | | | |
| Aspartic | 124.53 | 119.11 | 117.83 | 111.29 | |
| Glutamic | 204.12 | 203.09 | 191.54 | 195.97 | |
| Alanine | 47.71 | 45.08 | 43.77 | 41.24 | |
| Isoleucine | 48.49 | 47.71 | 44.69 | 42.40 | 28 |
| Phenylalanine | 55.52 | 58.58 | 52.91 | 49.19 | |
| Arginine | 76.37 | 77.57 | 74.17 | 71.08 | |
| Threonine | 41.36 | 37.53 | 37.83 | 35.94 | 34 |
| Proline | 55.41 | 51.95 | 53.26 | 50.58 | |
| Valine | 52.95 | 48.97 | 44.80 | 44.59 | 35 |
| Leucine | 86.06 | 90.39 | 81.37 | 78.11 | 66 |
| Histidine | 23.86 | 26.77 | 25.60 | 22.93 | 19 |
| Serine | 53.62 | 55.26 | 54.63 | 49.77 | |
| Glycine | 46.49 | 39.02 | 38.17 | 40.09 | |
| Tyrosine | 41.25 | 39.24 | 38.29 | 37.10 | |
| Lysine | 62.10 | 72.43 | 71.66 | 59.91 | 58 |
| | | | | | |
| Phe + tyr | 96.77 | 97.83 | 91.20 | 86.29 | 63 |

### Example 9

Processed cheese analogs were made using partially hydrolyzed BC-SPI, unhydrolyzed BC-SPI, Supro 760 (Com'l SPI A), Supro 710 (Com'l SPI C) or only rennet casein.

### Methods:

1. BC-SPI (45 g; 30% of the protein in the cheese formula) was added to water (414 g) at 50 degrees C and mixed thoroughly with a whisk until smooth (55 degrees C).
2. The pH of the solution was adjusted to 8.0 using 1 N NaOH.
3. Alcalase (0.18 g) was added to the mixture and stirred.
4. The mixture was placed in a Stephan Cooker at approximately 50 degrees C and mixed at 600 rpm for 20 minutes to allow time for limited hydrolysis of the soy proteins.
5. The mixture was then transferred to a beaker and heated to 60 degrees C for 10 minutes.
6. The slurry was added to a mixture of sodium citrate, sodium chloride, rennet casein and oil (50 degrees C) contained in a double boiler.
7. The mass was brought to 66 degrees C and lactic acid was added (pH of 5.65-5.75) and the mixture was stirred for 4 minutes, the time for the mass to reach 80 degrees C.
8. The hot mass was transferred to a Stephan Cooker (80 degrees C) and mixed at 600 rpm for 3 minutes and then poured into a plastic container, cooled for 5 minutes, sealed with a lid and stored in a refrigerator (4 degrees C).

Unhydrolyzed BC-SPI, Com'l SPI A, or Com'l isolate C were also used to make processed cheese analog by mixing the hydrated protein with the other ingredients in the double boiler and following steps 7-8 above. The formula in Table 12 indicates that the cheeses were 19% protein, 30% fat and 46% moisture. The moisture levels of these cheeses were higher than commercial product to make it feasible to compare the properties of wider range of soy protein ingredients in the model system, some of which would mix poorly at lower moisture levels.

**Results:** As shown in Table 12, the hydrolyzed commercial SPI (com'l SPI C) and the partially hydrolyzed BC-SPI had elastic textures and melted most similar to the cheeses containing only rennet casein as the protein source.

**Table 12 Cheese analog formula**

| **Ingredient** | **Weight grams** | **Protein grams** | **Fat grams** | **Moisture grams** |
|---|---|---|---|---|
| Rennet casein (N x 6.38 = 79.9% protein) | 108.0 | 86.29 | | |
| Soy protein (N x 5.71 = protein content) | 45.0 | 35.93 | | |
| Soybean oil | 184.8 | | 184.8 | |
| Sodium citrate | 15.0 | | | |
| Sodium chloride | 10.5 | | | |
| Water | 255.0 | | | |
| Lactic acid (80%) | 10.0 | | | |
| Total | 628.3 | | 184.8 | |
| Percent of formula | | 19.0 | 30.0 | 46.0 |

**Table 13. Results**

| **Sample** | **Appearance** | **pH** | **Moisture (%)** | **Melt (%)*** | |
|---|---|---|---|---|---|
| Partially hyd. BC-SPI | Elastic | 6.5 | 48.0 | | 47.6 |
| BC-SPI | Mealy | 5.89 | 48.2 | 39 | (0.8) |
| Com'l SPI A | Mealy | 5.86 | 47.2 | 41 | (3.4) |
| Com'l SPt C | Elastic | 5.92 | 47.7 | 65.5 | (1.7) |
| Rennet casein | Elastic | 5.93 | 48.8 | 76.2 | |

| | | | | | |
|---|---|---|---|---|---|
| *% decrease in height of 14 mm high cube heated in 100 degrees C oven for 15 minutes. Numbers in brackets show standard deviation for data on two days. | | | | | |

### Example 10

The preparation of new low methionine, high arginine containing BC-SPIs are conceived in the invention which are useful as nutrition and dietary supplements for modulating the total homocysteine level in plasma. BC-SPIs can be prepared by using conditions which limit disulfide interchange reactions between soy proteins (e.g. by using a reducing agent such as sodium bisulfite) and ultrafiltration to retain beta-conglycinin, glycinin and gamma-conglycinins. The lower molecular weight cysteine and methionine rich proteins will pass through the membrane in the permeate.

### Example 11

Lipoxygenase enzymes are known to cause off-flavor development in soy protein ingredients by catalyzing the oxidation of polyunsaturated fats. The lipoxygenase null trait of a soybean variety developed by Keisuke Kitamura were transferred to a U. S. food bean lacking two lipoxygenase genes and then further crossed with the high beta-conglycinin variety to create a low-flavor, high beta-conglycinin soybean varieties of the invention lacking at least two lipoxygenases.

## Claims

1. A soy protein isolate having a beta-conglycinin content greater than 40% of the protein and a glycinin content of less than 10% of the protein, wherein the sum of methionine and cysteine in the composition is greater than 24 mg/g protein, and further wherein
(a) the beta-conglycinin lacks alpha and alpha'-subunits; or
(b) the composition lacks one, two or three lipoxygenases.

2. The composition of claim 1, which is a composition that lacks one, two or three lipoxygenases.

3. The composition of claim 2, wherein the composition lacks one lipoxygenase.

4. The composition of claim 2, wherein the composition lacks two lipoxygenases.

5. The composition of claim 2, wherein the composition lacks three lipoxygenases.

6. Use of the composition of claim 1 or 2 as
(a) emsulsified meat;
(b) a soy cheese analog; or
(c) a liquid frozen coffee whitener.

## Patentansprüche

1. Sojaproteinisolat mit einem Beta-Conglycinin-Gehalt von mehr als 40% des Proteins und einem Glyciningehalt von weniger als 10% des Proteins, wobei die Summe von Methionin und Cystein in der Zusammensetzung größer als 24 mg/g Protein ist und wobei weiterhin
(a) das Beta-Conglycinin keine alpha- und alpha'-Untereinheiten aufweist; oder
(b) in der Zusammensetzung eine, zwei oder drei Lipoxygenasen fehlen.

2. Zusammensetzung gemäß Anspruch 1, bei der es sich um eine Zusammensetzung handelt, in der eine, zwei oder drei Lipoxygenasen fehlen.

3. Zusammensetzung gemäß Anspruch 2, wobei in der Zusammensetzung eine Lipoxygenase fehlt.

4. Zusammensetzung gemäß Anspruch 2, wobei in der Zusammensetzung zwei Lipoxygenasen fehlen.

5. Zusammensetzung gemäß Anspruch 2, wobei in der Zusammensetzung drei Lipoxygenasen fehlen.

6. Verwendung der Zusammensetzung gemäß Anspruch 1 oder 2 als
(a) emulgiertes Fleisch;
(b) Soja-Käseanalogon; oder
(c) flüssiger gefrorener Kaffeeweißer.

## Revendications

1. Isolat de la protéine de soja ayant une teneur en bêta-conglycinine supérieure à 40% de la protéine et une teneur en glycinine inférieure à 10% de la protéine, où la somme de la méthionine et de la cystéine dans la composition est supérieure à 24 mg/g de protéine, et où par ailleurs
(a) il manque des sous-unités alpha et alpha' à la bêta-conglycinine ; ou
(b) il manque une, deux ou trois lipoxygénase(s) à la composition.

2. Composition de la revendication 1, qui est une composition à laquelle il manque une, deux ou trois lipoxygénase(s).

3. Composition de la revendication 2, dans laquelle il manque une lipoxygénase à la composition.

4. Composition de la revendication 2, dans laquelle il manque deux lipoxygénases à la composition.

5. Composition de revendication 2, dans laquelle il manque trois lipoxygénases à la composition.

6. Utilisation de la composition de la revendication 1 ou 2 en tant que
(a) viande émulsifiée ;
(b) analogue de fromage de soja ; ou
(c) colorant à café congelé liquide.
